# EUROPEAN PATENT APPLICATION

(11) **EP 2 433 616 A1**
(43) Date of publication of application: **28.03.2012**
(21) Application number: 10777795.5
(22) Date of filing: 19.05.2010
(51) Int. Cl.: A61K 8/39, A61K 8/04, A61Q 5/02, A61Q 5/10, A61Q 5/12, A61Q 19/10

(54) **COSMETIC PREPARATION**

(30) Priority: 19.05.2009 JP 2009120828; 19.05.2009 JP 2009120829
(71) Applicant: Shiseido Company, Ltd., Chuo-ku Tokyo 104-8010 (JP)
(72) Inventor: SUGIYAMA, Yuki, Yokohama-shi Kanagawa 224-8558 (JP); WATANABE, Kei, Yokohama-shi Kanagawa 224-8558 (JP); NISHIJIMA, Yoshihito, Yokohama-shi Kanagawa 224-8558 (JP)
(74) Representative: Lippert, Stachow & Partner
(86) International application number: PCT/JP2010/058479
(87) International publication number: WO 2010/134562

(57) **Abstract**

An object of the invention is to provide a cosmetic composition that is not drippy during makeup removal, has good skin spreadability during use, has a refreshing feeling in use, and provides a high cleansing effect, and in particular, to provide a cleansing composition. The cosmetic composition according to the invention is **characterized by** comprising (A) an alkyl ethylene oxide surfactant having an HLB of 8 to 13, (B) 10 to 40 mass % of an oil component, and (C) water, wherein the composition is a discontinuous-micellar cubic liquid-crystal phase. Also the cosmetic composition is **characterized by** comprising the above composition as the outer phase and (B₂) an oil phase comprising an oil component, that is different from the oil component (B₁) captured in said discontinuous-micellar cubic liquid-crystal phase, as the inner phase.

## Description

### RELATED APPLICATIONS

This application claims the priority of Japanese Patent Application No. 2009-120828 filed on May 19, 2009 and Japanese Patent Application No. 2009-120829 filed on May 19, 2009, which are incorporated herein by reference.

### FIELD OF THE INVENTION

The present invention relates to a cosmetic composition, and in particular, relates to the improvement of the texture in use and cleansing effect.

### BACKGROUND OF THE INVENTION

In the past, an oil was blended into cleansing cosmetics and makeup-removing cosmetics for the removal of dirt on the skin and hair and for the removal of makeup cosmetics in order to dissolve and disperse dirt and makeup. There are O/W and W/O emulsion types, in which emulsion is achieved with an amphiphilic substance, and oil types, in which several kinds of oils are mixed. And more recently, there are aqueous types, in which oil is not blended and a large amount of surfactant having a moderate HLB is blended.

The O/W emulsion type makeup-removing cosmetics can be handled with wet hands and a refreshing texture in use can be obtained. However, the blending to makeup takes place after the coalescence of emulsion particles. Thus, there have been issues in that the penetration is slow and the cleansing power is weak. On the other hand, the W/O emulsion type and oil-type makeup removers have little issues in the blending to makeup and cleansing ability. However, there are serious issues in the texture in use, such as a residual oily feeling, after wiping with tissue or after washing the face. In the case of aqueous type makeup-removing cosmetics, there is no oily feeling and a fresh feeling by rinsing can be obtained. However, there has been a problem in that the cleansing power is not satisfactory.

In order to solve the above-described problems of makeup removers, a cleansing composition having a bicontinuous structure, in which the water phase and the oil phase form a continuous phase, was recently developed by the combined use of a specific nonionic surfactant, a low-viscosity oil, a water-soluble solvent, and water (for example, refer to Japanese Unexamined Patent Application Publication No. 2008-184413 and Japanese Unexamined Patent Application Publication No. 2004-217640). The cleansing property of the above-described composition is very high because it has the above-described continuous phase. However, a problem such as dripping takes place during makeup removal because it is a low-viscosity liquid.

In addition, a gel-like cleansing composition was developed, in which a hydrophilic nonionic surfactant, an anionic surfactant, a water-soluble compound having hydroxyl groups, a liquid oil, and water are contained in specific blending quantities (for example, refer to Japanese Unexamined Patent Application Publication No. H9-175936). The above-described composition, however, causes an oily film feeling and a sticky feeling during rinsing or after rinsing because a large amount of oil is blended; thus there is an issue in that the fresh feeling during use is not sufficient.

In order to improve the compatibility with makeup remover, a cosmetic composition containing a nonionic surfactant, water-soluble substance(s) having hydroxyl groups, a silicone oil, and water; and having a liquid crystal phase and/or an isotropic surfactant continuous phase, was developed (for example, refer to Japanese Unexamined Patent Application Publication No. 2000-256124). The above-described cosmetic composition generates a relatively less oily feeling on the skin during application and after rinsing. However, the cleansing action was not satisfactory. In addition, the spreadability on the skin and the blending to dirt were not satisfactory. Furthermore, the water compatibility was poor, and the above-described composition and dirt could not easily be removed because of water.

### DISCLOSURE OF THE INVENTION

### PROBLEM TO BE SOLVED BY THE INVENTION

The present invention was made in view of the above-described problems of the conventional art. An object is to provide a cosmetic composition that is not drippy during makeup removal, has good skin spreadability during use, has a refreshing feeling in use, and provides a high cleansing effect, and in particular, to provide a cleansing composition.

### MEANS TO SOLVE THE PROBLEM

The present inventors have diligently studied to solve the above-described problems. As a result, the present inventors have found that the composition wherein a discontinuous-micellar cubic liquid-crystal phase (I₁) is formed by blending a nonionic surfactant having an HLB of 8 to 13 and a large amount of oil and water, and the composition of oil-in-micellar cubic liquid-crystal phase (O/I₁)-type emulsion wherein the discontinuous-micellar cubic liquid-crystal phase constitutes the outer phase and additional oil is retained as emulsion particles (inner phase), are not drippy during makeup removal, have good spreadability on the skin, have a refreshing feeling in use, provide a high cleansing effect, and can be cleanly rinsed off because of good compatibility with water, thus leading to completion of the present invention.

That is, the first mode of the cosmetic composition of the present invention is a composition comprising (A) an alkyl ethylene oxide surfactant having an HLB of 8 to 13, (B) 10 to 40 mass % of an oil component, and (C) water, and is **characterized in that** the composition is a discontinuous-micellar cubic liquid-crystal phase.

In the cosmetic composition, it is preferable that the mass ratio of the component (A) and the component (C) is (A):(C) = 20:80 to 70:30.

In the cosmetic composition, it is preferable that the cosmetic composition is a cleansing composition.

In the cosmetic composition, it is preferable that the cleansing composition is a skin or hair cleansing composition.

In the cosmetic composition, it is preferable that the cleansing composition is a makeup cleansing base formula.

In the cosmetic composition, it is preferable to further comprise (D) a polyhydric alcohol and/or saccharide.

In the cosmetic composition, it is preferable that component (D) is polyethylene glycol and/or sorbitol.

The second mode of the cosmetic composition of the present invention is **characterized in that** a discontinuous-micellar cubic liquid-crystal phase comprising (A) an alkyl ethylene oxide surfactant having an HLB of 8 to 13, (B₁) an oil component, and (C) water is the outer phase and an oil phase comprising an oil component (B₂), which is different from the oil component (B₁) captured in the discontinuous-micellar cubic liquid-crystal phase, is contained as the inner phase.

In the cosmetic composition, it is preferable that the total mass of the (B) oil components ((B₁) + (B₂)) in the cosmetic composition is 20 to 80 mass % with respect to the total cosmetic composition.

In the cosmetic composition, it is preferable that the mass ratio of the component (A) and the component (C) is (A):(C) = 20:80 to 70:30.

In the cosmetic composition, it is preferable that the cosmetic composition is a cleansing composition.

In the cosmetic composition, it is preferable that the cleansing composition is a skin or hair cleansing composition.

In the cosmetic composition, it is preferable that the cleansing composition is a makeup cleansing base formula.

In the cosmetic composition, it is preferable to further comprise (D) a polyhydric alcohol and/or saccharide.

In the cosmetic composition, it is preferable that component (D) comprises one or more selected from the group consisting of 1,3-butylene glycol, dipropylene glycol, glycerin, polyethylene glycol, and sorbitol .

### EFFECT OF THE INVENTION

According to the present invention, a cosmetic composition that is not drippy and has good spreadability on the skin can be obtained. The cosmetic composition of the present invention has a high cleansing effect, and it can be cleanly rinsed off because of good compatibility with water. Thus, an excellent product can be provided especially as a cleansing composition.

### BRIEF DESCRIPTION OF THE DRAWINGS

Fig. 1 schematically shows a structure of the oil-in-micellar cubic liquid-crystal phase (O/I₁)-type emulsion composition.

Fig. 2 shows a pseudo-three component system phase diagram (water-surfactant-oil) for the components of Example 1-1, Comparative Example 1-1, and Comparative Example 1-2.

Fig. 3 shows the phase state of the composition when an oil component was added to the mixture wherein a surfactant and ion-exchanged water or a polyhydric alcohol aqueous solution were mixed in a mass ratio of 38:62 (surfactant:aqueous solution).

Fig. 4 shows a pseudo-three component system phase diagram (water-surfactant-oil) for the components of Example 2-1, Comparative Example 2-1, and Comparative Example 2-2.

Fig. 5 shows the makeup removing effect of the cosmetic composition of the present invention and that of conventional cleansing cosmetics.

### BEST MODE FOR CARRYING OUT THE INVENTION

### DESCRIPTION OF THE PREFERRED EMBODIMENT

Hereinafter, the embodiment of the present invention will be described in detail.

The first mode of the cosmetic composition of the present invention is a composition wherein the composition comprises (A) an alkyl ethylene oxide surfactant within a specific range of HLB, (B₁) a specific amount of oil component, and (C) water, and a discontinuous-micellar cubic liquid-crystal phase (I₁) is formed.

The second mode of the cosmetic composition of the present invention is an oil-in-micellar cubic liquid-crystal phase (O/I₁)-type emulsion composition wherein the discontinuous-micellar cubic liquid-crystal phase composition is the outer phase, and an oil phase comprising (B₂) an oil component that is different from the (B₁) specific amount of oil component captured in the liquid crystal phase is the inner phase.

At first, respective components will be explained.

### Alkyl ethylene oxide surfactant

(A) alkyl ethylene oxide surfactant used in the present invention includes polyoxyethylene lauryl ether, polyoxyethylene cetyl ether, polyoxyethylene oleyl ether, polyoxyethylene stearyl ether, polyoxyethylene isocetyl ether and polyoxyethylene isostearyl ether. One or more of these surfactants may be arbitrarily selected for use in a cosmetic composition of the present invention.

It is preferable that the HLB of the alkyl ethylene oxide surfactant of the present invention is 8 to 13 and especially preferable to be 8 to 10. It is generally known that the solubility into water and the solubility into oil are balanced when the HLB is 7. That is, the preferable surfactant of the present invention is a surfactant whose oil/water solubility is near medium to slightly hydrophilic. If the HLB is 8 or lower, discontinuous-micellar cubic liquid crystals cannot be obtained, the good cleanser rinsability during rinsing cannot be achieved, and the spreadability and cleansing effect are also not satisfactory. If the HLB exceeds 13, discontinuous-micellar cubic liquid crystals containing a large amount of oil cannot be obtained, and a good cleansing effect cannot be achieved.

The alkyl ethylene oxide surfactant commercially available includes EMALEX 705 (polyoxyethylene (5 mol) lauryl ether, HLB: 10), EMALEX 707 (polyoxyethylene (7 mol) lauryl ether, HLB: 10), EMALEX 709 (polyoxyethylene (9 mol) lauryl ether, HLB: 11), EMALEX 712 (polyoxyethylene (12 mol) lauryl ether, HLB: 13), EMALEX 105 (polyoxyethylene (5 mol) cetyl ether, HLB: 8), EMALEX 107 (polyoxyethylene (7 mol) cetyl ether, HLB: 10), EMALEX 110 (polyoxyethylene (10 mol) cetyl ether, HLB: 11), EMALEX 112 (polyoxyethylene (12 mol) cetyl ether, HLB: 12), EMALEX 115 (polyoxyethylene (15 mol) cetyl ether, HLB: 13), EMALEX 506 (polyoxyethylene (6 mol) oleyl ether, HLB: 8), EMALEX 508 (polyoxyethylene (8 mol) oleyl ether, HLB: 10), EMALEX 510 (polyoxyethylene (10 mol) oleyl ether, HLB: 11), EMALEX 512 (polyoxyethylene (12 mol) oleyl ether, HLB: 11), EMALEX 515 (polyoxyethylene (15 mol) oleyl ether, HLB: 12), EMALEX 603 (polyoxyethylene (3 mol) stearyl ether, HLB: 10), EMALEX 605 (polyoxyethylene (5 mol) stearyl ether, HLB: 8), EMALEX 606 (polyoxyethylene (6 mol) stearyl ether, HLB: 8), EMALEX 611 (polyoxyethylene (11 mol) stearyl ether, HLB: 11), EMALEX 615 (polyoxyethylene (15 mol) stearyl ether, HLB: 12), EMALEX 1605 (polyoxyethylene (5 mol) isocetyl ether, HLB: 8), EMALEX 1610 (polyoxyethylene (10 mol) isocetyl ether, HLB: 11), EMALEX 1615 (polyoxyethylene (15 mol) isocetyl ether, HLB: 13), EMALEX 1805 (polyoxyethylene (5 mol) isostearyl ether, HLB: 8), EMALEX 1810 (polyoxyethylene (10 mol) isostearyl ether, HLB: 11), EMALEX 1815 (polyoxyethylene (15 mol) isostearyl ether, HLB: 12) and EMALEX 1820 (polyoxyethylene (20 mol) isostearyl ether, HLB: 13), manufactured by Nihon Emulsion Co., Ltd.

In the present invention, the above-described polyoxyethylene alkyl ether surfactant is especially preferably used. However, so far as discontinuous-micellar cubic liquid crystals can be formed, an ester-type surfactant such as PEG-8 glyceryl isostearate, for example, can be used as the alkyl ethylene oxide surfactant of the present invention.

The blending quantity of the component (A), an alkyl ethylene oxide surfactant having an HLB of 8 to 13, is preferably 0.1 to 60 mass % with respect to the total amount of the composition, especially preferably 0.5 to 50 mass %, and more preferably 0.5 to 30 mass %. If the blending quantity is less than 0.1 mass %, discontinuous-micellar cubic liquid crystals cannot be formed, and the desired phase state may not be obtained. If the blending quantity exceeds 60 mass %, irritation to the skin may become strong.

Examples of the (B) oil component include hydrocarbon oils, higher fatty acids, higher alcohols, synthetic ester oils, liquid fats, and silicone oils, which are normally used in cosmetics, quasi-drug products, etc, and one or more kinds of oils can be used.

Hydrocarbon oils include isododecane, isohexadecane, isoparaffin, paraffin, liquid paraffin, squalane, squalene and ceresin.

Higher fatty acids include lauric acid, myristic acid, palmitic acid, stearic acid, behenic acid, oleic acid, undecylenic acid, tall oil acid, isostearic acid, linoleic acid, linolenic acid, eicosapentanoic acid (EPA) and docosahexaenoic acid (DHA).

Higher alcohols include straight chain alcohols such as lauryl alcohol, cetyl alcohol, stearyl alcohol, behenyl alcohol, myristyl alcohol, oleyl alcohol and cetostearyl alcohol; and branched-chain alcohols such as monostearyl glycerin ether (batyl alcohol), 2-decyltetradecanol, lanolin alcohol, cholesterol, phytosterol, hexyldodecanol, isostearyl alcohol and octyldodecanol.

Synthetic ester oils include isopropyl myristate, cetyl octanoate, cetyl 2-ethylhexanoate, octyldodecyl myristate, isopropyl palmitate, butyl stearate, hexyl laurate, myristyl myristate, decyl oleate, dimethyl octanoic acid hexyldecyl, cetyl lactate, myristyl lactate, acetylated lanolin, isocetyl stearate, isocetyl isostearate, cholesteryl 12-hydroxystearate, ethylene glycol di-2-ethylhexanoate, dipentaerythritol fatty acid ester, N-alkyl glycol monoisostearate, neopentyl glycol dicaprate, diisostearyl malate, glyceryl di-2-heptylundecanoate, trimethylol propane tri(2-ethylhexanoate), trimethylol propane triisostearate, pentaerythritol tetra(2-ethylhexanoate), glyceryl tri(2-ethylhexanoate), glyceryl trioctanoate, glyceryl triisopalmitate, trimethylolpropane triisostearate, 2-ethylhexyl palmitate, glycerin trimyristate, glyceride tri-2-heptylundecanoate, castor oil fatty acid methylester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate and triethyl citrate.

Liquid fats include linseed oil, camellia oil, macedamia nut oil, corn oil, mink oil, olive oil, avocado oil, sasanqua oil, castor oil, safflower oil, jojoba oil, sunflower seed oil, almond oil, rapseed oil, sesame oil, soybean oil, peanut oil, triglyceride, glycerol trioctanoate glycerol trioctanoate and glycerol triisopalmitate.

Silicone oils include chain silicones such as dimethylpolysiloxane, methylphenylpolysiloxane and methylhydrogenpolysiloxane; and cyclic silicones such as octamethylcyclotetrasiloxane, decamethylcyclopentasiloxane and dodecamethylcyclohexasiloxane.

The blending quantity of the oil component, when a discontinuous-micellar cubic liquid crystal composition (I₁) is to be obtained, is preferably in the range of 10 to 40 mass % with respect to the total amount of the composition and more preferably 15 to 30 mass %. In this case, if the blending quantity of the oil component is less than 10 mass %, a satisfactory cleansing effect may not be obtained. If the blending quantity exceeds 40 mass %, the desired association state, namely, discontinuous-micellar cubic liquid crystals may not be obtained.

When a composition (O/I₁), wherein the discontinuous-micellar cubic liquid crystals form the outer phase, and an oil component that is different from the oil component in the micelle of the liquid crystals is contained as the inner phase of the system, is to be obtained, the blending quantity of the oil component is preferably in the range of 20 to 80 mass % with respect to the total amount of the composition and more preferably 40 to 80 mass %. In this case, if the blending quantity of the oil component is less than 20 mass %, a satisfactory cleansing effect cannot be obtained and the spreadability during use may not be good. If the blending quantity exceeds 80 mass %, the composition may lack in stability. In such a composition, the amount of the oil (B₁) captured in the micelle of the discontinuous-micellar cubic liquid-crystal phase is preferably 5 to 30 mass % from the standpoint of cleansing property and good spreadability.

The blending quantity of (C) water used in the present invention is not limited in particular. However, the blending quantity needs to be suitably adjusted, depending upon the kinds and blending quantities of (A) and (B) used in the preparation of the cosmetic composition, within the range in which discontinuous-micellar cubic liquid crystals can be formed. The blending range is preferably (A):(C) = 20:80 to 70:30 and more preferably 20:80 to 45:55. If component (A) is blended in excess of this range, the stickiness may be generated during use and it may not be desirable from the standpoint of safety. If the blending quantity is below this, discontinuous-micellar cubic liquid crystals may not be formed.

### Polyhydric alcohol and/or saccharide

In the cosmetic composition of the present invention, it is preferable to additionally blend a polyhydric alcohol and/or a saccharide as the component (D).

By blending a polyhydric alcohol and/or saccharide, the elasticity of the discontinuous-micellar cubic liquid-crystal phase of the cosmetic composition of the present invention is lowered, and a soft and extendable texture in use can be provided to the composition.

Furthermore, by blending the above-described component into the discontinuous-micellar cubic liquid-crystal phase composition (I₁), the amount of the oil component captured into the spherical micelle, in the micellar cubic liquid crystal phase of the cosmetic composition of the present invention, may be significantly increased. When the amount of the oil component contained in the cosmetic composition is increased, the functionality (cleansing power) of the present invention as a makeup cleansing composition etc. is drastically improved as expected.

That is, the discontinuous-micellar cubic liquid-crystal (I₁) phase composition wherein a polyhydric alcohol and/or a saccharide is blended has not only a desirable texture in use (good spreadability) but also excellent functionality (cleansing power) in some cases.

In the composition (O/I₁), wherein the discontinuous-micellar cubic liquid-crystal phase is the outer phase and an oil component different from the oil component in the micelle of the liquid crystal is contained as the inner phase of the system, when the above-described component (D) is added to the continuous phase of (C) an aqueous component, the refractive index of the discontinuous-micellar cubic liquid-crystal phase (namely, the outer phase), wherein a spherical-micellar cubic is formed in the continuous phase, increases. Thus, the refractive index difference from the oil phase (B₂) formed of (B) an oil component decreases. The decrease in the refractive index difference is associated with an increase in the transparency of the composition. Therefore, while the cosmetic composition of the present invention based on the O/I₁ emulsion normally has a cloudy creamy form, a high-transparency gel-like appearance can be obtained by blending the component (D).

The intended extent of the above-described texture in use and transparency may be adjusted by the combination of the used components (D) and the blending quantities, depending upon the application and the intended use of the present invention, so that the desired effect is achieved.

The polyhydric alcohols and sugars used in the present invention are not limited in particular so far as they are normally used in cosmetics and quasi-drug products.

Polyhydric alcohols include dihydric alcohols such as dipropylene glycol, 1,3-butylene glycol, ethylene glycol, trimethylene glycol, 1,2-butylene glycol, tetramethylene glycol, 2,3-butylene glycol, pentamethylene glycol, 2-butene-1,4-diol, hexylene glycol, octylene glycol; trihydric alcohols such as glycerin and trimethylolpropane; tetrahydric alcohols such as diglycerin, 1,2,6-hexanetriol and pentaerythritol; pentahydric alcohols such as xylitol and triglycerin; hexahydric alcohols such as sorbitol and mannitol; polyhydric alcohol polymers such as diethylene glycol, dipropylene glycol, triethylene glycol, polypropylene glycol, tetraethylene glycol, diglycerin, triglycerin, tetraglycerin and polyglycerin; divalent alcohol alkyl ethers such as ethylene glycol monomethyl ether, ethylene glycol monoethyl ether, ethylene glycol monobutyl ether, ethylene glycol monophenyl ether, ethylene glycol (mono?)hexyl ether, ethylene glycol mono2-methylhexyl ether, ethylene glycol isoamyl ether, ethylene glycol (mono?)benzyl ether, ethylene glycol (mono)isopropyl ether, ethylene glycol dimethyl ether, ethylene glycol diethyl ether and ethylene glycol dibutyl ether; dihydric alcohol alkyl ethers such as diethylene glycol monomethyl ether, diethylene glycol monoethyl ether, diethylene glycol monobutyl ether, diethylene glycol dimethyl ether, diethylene glycol diethyl ether, diethylene glycol dibutyl ether, diethylene glycol methylethyl ether, triethylene glycol monomethyl ether, triethylene glycol monoethyl ether, propylene glycol monomethyl ether, propylene glycol monoethyl ether, propylene glycol monobutyl ether, propylene glycol (mono?)isopropyl ether, dipropylene glycol (mono?)methyl ether, dipropylene glycol (mono?)ethyl ether and dipropylene glycol (mono?)butyl ether; dihydric alcohol ether ester such as ethylene glycol monomethyl ether acetate, ethylene glycol monoethyl ether acetate, ethylene glycol monobutyl ether acetate, ethylene glycol monophenyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate, propylene glycol monopropyl ether acetate and propylene glycol monophenyl ether acetate; glycerin monoalkyl ethers such as chimyl alcohol, selachyl alcohol and batyl alcohol; sugar alcohols such as maltotriose, mannitol, sucrose, erythritol, glucose, fructose, starch sugar, maltose and starch sugar reduced alcohol; glysolid; tetrahydrofurfuryl alcohol; POE tetrahydrofurfuryl alcohol; POP butyl ether; POP POE butyl ether; tripolyoxypropylene glycerin ether; POP glycerin ether; POP glycerin ether phosphoric acid; POP POE pentaerythritol ether; polyglycerin; polyoxyethylene methyl glucoside (Glucam E-10); and polyoxypropylene methyl glucoside (Glucam P-10).

Sugars include monosaccharides, oligosaccharides and polysaccharides.

Monosaccharides include trioses such as D-glyceraldehyde and dihydroxyacetone; tetroses such as D-erythrose, D-erythrulose, D-threose and etythritol; pentoses such as L-arabinose, D-xylose, L-lyxose, D-arabinose, D-ribose, D-ribulose, D-xylulose and L-xylulose; hexoses such as D-glucose, D-talose, D-psicose, D-galactose, D-fructose, L-galactose, L-mannose and D-tagatose; heptoses such as aldoheptose and heprose; octoses such as octrose; deoxysugars such as 2-deoxy-D-ribose, 6-deoxy-L-galactose and 6-deoxy-L-mannose; aminosugars such as D-glucosamine, D-galactosamine, sialic acid, aminouronic acid and muramic acid; and uronic acids such as D-glucuronic acid, D-mannuronic acid, L-guluronic acid, D-galacturonic acid and L-iduronic acid.

oligosaccharides include sucrose, gentianose, umbelliferose, lactose, planteose, isolychnose series, alpha,alpha-trehalose, raffinose, lychnose series, umbilicin, stachyose and verbascose.

Polysaccharides include cellulose, quince seeds, starch, galactan, dermatan sulfate, glycogen, gum arabic, heparan sulfate, gum tragacanth, keratan sulfate, chondroitin, xanthane gum, guar gum, dextran, kerato sulfate, locust bean gum and succinoglycan.

In the present invention, one or more from the above-described polyhydric alcohols and/or saccharides can be selected for use.

In the discontinuous-micellar cubic liquid-crystal (I₁) phase composition, the use of a trihydric or higher polyhydric alcohol is especially preferable. Furthermore, polyethylene glycol and/or sorbitol is suitable as the component (D) from the standpoint that they further decrease the elasticity of the micellar cubic liquid crystal phase and effectively increase the blending quantity of the oil component. Therefore, when such a composition is produced, the component (D) is preferably determined so that one or more selected from the above-described substances are contained.

When the component (D) is blended in the discontinuous-micellar cubic liquid-crystal (I₁) phase composition, the mass ratio with the component (C) is preferably (C):(D) = 10:90 to 60:40. If the deviation from the above-described mass ratio takes place in such a composition, the desired effect due to the blending of component (D) may not be obtained.

On the other hand, in the (O/I₁) composition, wherein the discontinuous-micellar cubic liquid-crystal phase is the outer phase and an oil component different from the oil component in the micelle of the liquid crystal is contained as the inner phase of the system, the use of trihydric or higher polyhydric alcohols is especially preferable. Furthermore, it is preferable that the component (D) comprises one or more selected from the group consisting of 1,3-butylene glycol, dipropylene glycol, glycerin, polyethylene glycol, and sorbitol.

When the component (D) is blended in such a composition, the mass ratio with the component (C) is preferably in the range of (C):(D)=10:90 to 60:40. If the mass ratio is in this range, the transparency of the composition can be satisfactorily adjusted. However, if the ratio of the component (D) relative to the component (C) is less than 10:90, the transparency generation effect may not be obtained.

In particular, by allowing the ratio to be (C):(D)=80:20 to 50:50, the spreadability of the composition can be improved in addition to the generation of transparency.

In the cosmetic composition of the present invention, so far as the effect of the present invention is not undermined, normally usable substances can be blended, as necessary, in addition to the above-described essential components.

Examples of moisturizing agent include 1,3-butylene glycol, polyethylene glycol, propylene glycol, dipropylene glycol, hexylene glycol, glycerin, diglycerin, xylitol, maltitol, maltose and D-mannitol.

Examples of water-soluble polymer include plant polymers such as gum arabic, carrageenan, pectin, agar, quince seeds (marmelo), starch, algal colloids (brown alga extract); microbial polymers such as dextran and pullulan; animal polymers such as collagen, casein and gelatin; starch polymers such as carboxymethyl starch and methyl hydroxypropyl starch; alginate polymers such as sodium alginate; vinyl polymers such as carboxy vinyl polymer (CARBOPOL); polyoxyethylene polymers; polyoxyethylene polyoxypropylene copolymer; acrylic polymers such as sodium polyacrylate and polyacrylamide; inorganic water-soluble polymers such as bentonite, magnesium aluminum silicate and lapnite.

Examples of ultraviolet absorber include benzoic acid ultraviolet absorbers such as para-aminobenzoic acid; anthranilic acid ultraviolet absorbers such as methyl anthranilate; salicylic acid ultraviolet absorbers such as octyl salicylate and phenyl salicylate; cinnamic acid ultraviolet absorbers such as isopropyl para-methoxy cinnamate, octyl para-methoxy cinnamate and glyceryl dipara-methoxy cinnamate mono-2-ethyl hexanoate; benzophenone ultraviolet absorbers such as 2,4-dihydroxy benzophenone, 2-hydroxy-4-methoxy benzophenone and 2-hydroxy-4-methoxy benzophenone-5-sulfonic acid; urocanic acid; 2-(2'-hydroxy-5'-methylphenyl) benzotriazole; and 4-tert-butyl-4'-methoxy benzoylmethane.

Examples of sequestering agent include edetate sodium, sodium metaphosphate and phosphoric acid.

Examples of antioxidant include ascorbic acid, alpha-tocopherol, dibutylhydroxytoluene and butylhydroxyanisol.

Examples of medicinal agent include vitamins such as vitamin A oil, retinol, retinol palmitate, inositol, pyridoxine hydrochloride, benzyl nicotinate, nicotinic-acid amide, DL-alpha-tocopherol nicotinate, magnesium ascorbyl phosphate, ascorbic acid 2-glucoside, vitamin D2 (ergocalciferol), L-ascorbic acid DL-alpha-tocopherol phosphoric acid diester potassium salt, DL-alpha-tocopherol, DL-alpha-tocopheryl acetate, pantothenic acid and biotin; anti-inflammatory agents such as allantoin and azulene; whitening agents such as arbutin; astringents such as zinc oxide and tannic acid; sulfur; lysozyme chloride; pyridoxine hydrochloride; and gamma-orizanol.

The above medicinal agents may be used not only in their free states but also in the form of salts of acid or base when they can form such salts or in the form of esters when they have a carboxylic group.

In the cosmetic composition of the present invention, it is necessary that a discontinuous-micellar cubic liquid-crystal phase is formed.

That is, the association state formed by a mixture of surfactant/oil component/water is broadly classified into microemulsions and liquid crystals. When excess water or oil is added thereto, a water-in-oil type emulsion composition or an oil-in-water type emulsion composition may be formed.

As microemulsions,
[1] micelle aqueous solution (L₁),
[2] reverse-micellar oil solution (L₂), and
[3] bicontinuous microemulsion (L₃)
are well known.

In the micelle aqueous solution, the surfactant concentration and oil concentration are low; therefore, the cleansing effect is poor. In the reverse-micellar oil solution, the viscosity is low and it is drippy, and the rinsability with water is slightly poor because of its high lipophilicity. The bicontinuous microemulsion has a bicontinuous structure of water and oil. Therefore, the cleansing property and rinsability are very good. However, there is an issue of dripping during makeup removal because it is a low-viscosity liquid.

The emulsion composition is very common, and many cosmetics are classified to this. However, most of them are cloudy as represented by cream and milky lotion. When the oil-in-water type emulsion composition is used as cleansing composition, the cleansing power is weak. On the other hand, when the water-in-oil type emulsion composition is used as cleansing composition, the rinsability is not good, and a fresh feeling in use cannot be obtained.

As liquid crystal structures,
[I] micellar cubic (discontinuous cubic) liquid crystal phase (I₁)
[II] hexagonal liquid crystal phase (H₁)
[III] bicontinuous cubic liquid crystal (V₁)
[IV] lamellar liquid crystal phase (L_{α})
[V] bicontinuous cubic liquid crystal (V₂)
[VI] reverse-hexagonal liquid crystal phase (H₂)
[VII] reverse-micellar cubic (discontinuous cubic) liquid crystal phase (I₂)
are well known.

Among them, the lamellar liquid crystal phase (L_{α}) often has a high concentration of surfactant; thus irritation to the skin is observed. The reverse-hexagonal liquid crystal phase and reverse-micellar cubic liquid crystal phase have high lipophilicity; thus they lack rinsability. The hexagonal liquid crystal phase (H₁) has poor spreadability during use.

The present inventors focused on a discontinuous cubic (micellar cubic) liquid crystal phase to obtain a makeup cleansing base formula with excellent water rinsability, non-drippy during makeup removal, and with good spreadability during use.

In the discontinuous cubic liquid crystal phase, either the hydrophobic region or the hydrophilic region in the liquid crystal structure takes a continuous structure, and the other region takes a spherically closed structure. In the micellar cubic liquid crystal phase (I₁), the hydrophilic region of the discontinuous cubic takes a continuous structure, and the hydrophobic region takes a spherically closed structure. That is, surfactant molecules form aggregates with their hydrophobic regions facing inside and the hydrophilic regions facing outside. These aggregates further form a liquid crystal structure by regular three-dimensional alignment.

However, it has been considered to be difficult to blend a high concentration of oil into the discontinuous-micellar cubic liquid crystals.

Thus, the present inventors have developed a cosmetic composition that was prepared with an alkyl ethylene oxide surfactant having a medium HLB to a slightly high HLB (from moderately polar to slightly hydrophilic), oil components, and water so that discontinuous-micellar cubic liquid crystals wherein the amount of solubilized oil is dramatically improved can be obtained.

The discontinuous-micellar cubic liquid crystals have high viscoelasticity because of the structure. Therefore, it is possible to retain the oil as emulsion particle droplets by adding more oil than the threshold concentration at which the most oil can be captured in the micellar cubic liquid crystal structure. That is, an oil-in-cubic liquid crystal (O/I₁)-type emulsion composition can be obtained. When we consider the use of the oil-in-cubic liquid crystal-type emulsion composition, wherein the discontinuous-micellar cubic liquid-crystal phase is used as the outer phase and an additional oil component (B₂) is retained as emulsion particles in the inner phase, as a cleansing composition, a large amount of oil needs to be contained to increase the cleansing power. However, there is a limit in the amount of oil (B₂) that can be stably retained as emulsion particles. Therefore, it is preferable that a large amount of oil (B₁) is also contained in the cubic liquid crystal structure, which is the outer phase, to obtain an emulsion composition containing a larger amount of oil. That is, in order to increase the total amount of oil B (= B₁ + B₂), it is important to increase not only B₂ but also B₁. As described above, however, it has been considered to be difficult to blend a high concentration of oil into the discontinuous-micellar cubic liquid crystals.

Thus, the present inventors formed a discontinuous-micellar cubic liquid-crystal phase (I₁), wherein a cubic structure of micelles of an alkyl ethylene oxide surfactant having a medium HLB to a slightly high HLB (from moderately polar to slightly hydrophilic) was arranged in the water (aqueous component) continuous phase. The present inventors also developed an oil-in-micellar cubic liquid-crystal phase (O/I₁)-type emulsion composition wherein the liquid crystal phase was used as the outer phase and oil was retained as the inner phase.

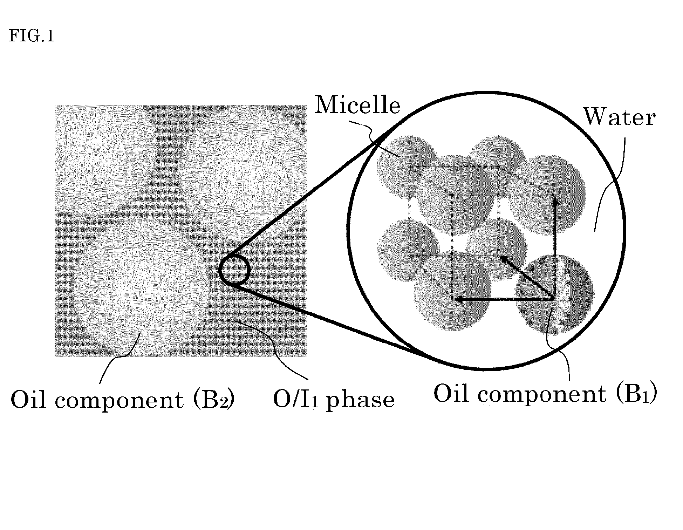

Fig. 1 illustrates a discontinuous-micellar cubic liquid crystal structure and an oil phase in the micellar cubic liquid crystal phase. As shown in Fig. 1, there are two kinds of oil (oil components) in the composition of the present invention: the oil contained in the individual micelle (B₁) and emulsion particles (B₂) dispersed in the liquid crystals. In the present invention, the conditions that increase the amount of these solubilized oils were identified, and the high cleansing power and the improvement of texture in use were realized.

It is preferable to form an oil-in-micellar cubic liquid-crystal phase (O/I₁)-type emulsion composition, which is formed from the cosmetic composition of the present invention, through the formation of the I₁ phase composition. For example, I₁ is initially formed when the above-described constituent components (A) to (C) are mixed and the component (B) is gradually added. By the further addition of the component (B), the excess component (B) is dispersed in the liquid crystals and O/I₁ is formed.

In the following, the determination method of a discontinuous-micellar cubic liquid-crystal phase will be described.

### Sample preparation

(1) A surfactant, ion-exchanged water, and oil are weighed into a sample tube and stirred with a strong shearing force.

(2) When the sample of (1) cannot be stirred well, the temperature is increased to near 70 °C and the stirring is performed. After allowing it to return to room temperature, the stirring is repeated.

(3) Gas bubbles are removed by centrifuging it for a sufficient time.

(4) Whether the obtained sample is a discontinuous-micellar cubic liquid-crystal phase can be determined by the methods "determination by appearance", "polarization microscope observation and X-ray structural analysis", "preparation of phase equilibrium diagram", "electrical conductivity measurement ", "self-diffusion coefficient measurement by NMR", "electron microscope observation of replicas prepared by the freeze-fracture method", etc. Any method can be used for determination.

### Determination by appearance

In the determination by appearance, the discontinuous-micellar cubic liquid-crystal phase is a transparent to translucent one-phase region and optically isotropic. The determination of optical anisotropy is possible by confirming the presence of light transmission when a sample is retained between two polarizing plates with a phase difference of 90°.

The oil-in-micellar cubic liquid-crystal phase (O/I₁)-type emulsion composition is normally opaque. However, the adjustment to the transparency close to that of the I₁ phase is possible by the addition of a polyhydric alcohol and/or a saccharide.

In such a case, I₁ and O/I₁ can be distinguished by the distance between cubic micelles d, which is determined by X-ray structural analysis (SAXS). That is, when the oil concentration of the system is varied, the region where the value d changes is I₁, and the region where the value does not change is determined to be O/I₁.

### Polarization microscope observation and X-ray structural analysis

According to polarization microscope observation and X-ray structural analysis, when an anisotropic band pattern is observed and the scattering peaks corresponding to the layer spacing appear, it is considered to be a lamellar liquid crystal phase. When an anisotropic stripe pattern is observed and sharp scattering peaks appear at the spacing period of hexagonal rod-like micelles, it is considered to be a hexagonal liquid crystal phase. When it is a dark field in polarization microscope observation and the spots of cubic symmetry appear, it is considered to be a cubic liquid crystal phase.

### Preparation of phase equilibrium diagram

In the preparation of a phase equilibrium diagram, the identification is possible by the characteristics that when a phase equilibrium diagram for the three component system consisting of water/oil component/surfactant is prepared, the concentration range wherein a discontinuous-micellar cubic liquid-crystal phase is formed is in the neighboring region of a hexagonal liquid crystal phase. However, the characteristics are different depending upon the constituent system (components).

### Electrical conductivity measurement

In the electrical conductivity measurement, the liquid crystal phase has characteristic electrical properties due to the fluidity, structural continuity, and anisotropy of the respective phases. Therefore, the information of phases can be obtained by carrying out conductivity measurements under suitable conditions. For a water-continuous liquid-crystal structure (a discontinuous-micellar cubic liquid-crystal phase corresponds to this), a high electrical conductivity can be observed. Therefore, for example, the distinction from a bicontinuous cubic liquid crystal phase having about 1/3 the electrical conductivity is possible.

### Measurement of self-diffusion coefficient by NMR

The measurement method of self-diffusion coefficient by NMR is described in detail by Lindman et al. in J. Colloid Interface Sci. 1981, 83, 569, and the like.

### Electron microscope observation of replicas prepared by freeze-fracture method

An image of a discontinuous-micellar cubic liquid-crystal phase can be obtained in the electron microscope observation of the phase sample prepared by a freeze-fracture method. Based on this image, the distinction is easy from the aggregate images obtained for a hexagonal liquid crystal phase, lamellar liquid crystal phase, and other phases. This method is described in detail in the literature by Imae et al., Colloid polym. Sci. 1994, 272, 604.

Thus, when an optically isotropic high-transparency viscoelastic body, wherein the mass ratio of the composition component (A) alkyl ethylene oxide surfactant and the component (C) water is 20:80 to 70:30 and a suitable amount of oil is contained, is formed, it can be confirmed to be a discontinuous-micellar cubic liquid-crystal phase by the above-described various methods.

The HLB of the surfactant used in the present invention is 8 to 13. Because the surfactant is hydrophilic, it is obtained on the phase diagram as the region of a discontinuous-micellar cubic liquid-crystal phase.

The region where the surfactant concentration is lower than this will become a completely cloudy O/W emulsion region judging from HLB. In the O/W emulsion region, the oil is dispersed in the size of about a few µm; therefore, a light is scattered at all the wavelengths and a white color is shown. In addition, the desired cleansing effect cannot be achieved because the continuous outer phase is water.

The cosmetic composition of the present invention can be widely used in various applications as cleansing compositions, skin care cosmetics, hair cosmetics, and makeup cosmetics. In addition, they can be widely used for various applications traditionally used as cleansing compositions. Examples include shampoo, body cleanser, facial cleanser, wet tissue (for cleansing), wet tissue (for face cleansing), and makeup remover (for mascara, eye shadow, and foundation). Examples of skin care cosmetics include massage gel, body gel, and face pack. Examples of hair cosmetics include hair treatment, rinse, hair styling preparation, and hair color. Because the rinsability of the cosmetic composition of the present invention is good, it is also desirable as a rinsable cosmetic.

In addition, the present invention can be applied to the types of products wherein the desired phase state is obtained by adding water from the outside, for example, by facial cleansing.

Hereinafter, the present invention will be described in further detail with reference to the examples. However, the present invention is not limited by these examples. The blending quantity is expressed in mass %, unless otherwise specified, with respect to the system in which the component is blended.

### EXAMPLES

Firstly, the present inventors investigated the discontinuous-micellar cubic liquid-crystal (I₁) phase composition. Initially, the evaluation methods used in the present examples will be explained.

### <Association state>

The association state of the cosmetic composition of the present invention was evaluated. The evaluation methods for determination are the above-described appearance observation, polarization microscope observation, X-ray structural analysis, and the preparation of a phase equilibrium diagram.
I₁: The association state is a discontinuous-micellar cubic liquid-crystal phase.
L_{α}: The association state is a lamellar liquid crystal phase.
H₁: The association state is a hexagonal liquid crystal phase.
L₂: The association state is a reverse-micellar oil solution phase.

### <Cleansing effect >

In order to determine the cleansing effect of the cosmetic composition of the present invention, the following cleansing effect tests were carried out.

### "Preparation of artificial dirt"

Artificial dirt 1: Vaseline containing the red pigment Sudan III and stearic acid were mixed in a ratio of 1:1 at 80 °C and cooled naturally.
Artificial dirt 2: 30% of hydrophobized powder was further added to the artificial dirt 1 and mixed at 80 °C and cooled naturally.

### "Cleansing effect evaluation method"

A region of 2 cm ×2 cm was beforehand set on the artificial leather, and 0.1 g of artificial dirt was uniformly applied. In a 100 mL screw vial, 50 mL of a cosmetic composition was placed, the artificial leather on which artificial dirt was applied was immersed into the cleansing solution, and it was vigorously shaken for 5 minutes. Then, the artificial leather was gently pulled up, and the residual amount of the artificial dirt was determined with a color difference meter.

### Determination criteria for "cleansing effect"

⊚ : The residual amount of the artificial dirt is 5% or lower.
○ : The residual amount of the artificial dirt is 20% or lower.
△ : The residual amount of the artificial dirt is 50% or lower.
× : The residual amount of the artificial dirt exceeds 50%.

### <Absence of dripping>

In order to determine the usability of the cosmetic composition of the present invention, the "dripping" property was determined by eight professional panelists. Determination criteria for "dripping"
○ : 7 to 8 panelists out of 8 panelists answered that there was no dripping during use and the usability was good.
△ : 5 to 6 panelists out of 8 panelists answered that there was no dripping during use and the usability was good.
× : 4 or more panelists out of 8 panelists answered that there was dripping during use and the usability was not good.

### <Good spreadability>

In order to determine the usability of the cosmetic composition of the present invention, the "spreadability during use" was determined by eight professional panelists. Determination criteria for "good spreadability"
○ : 7 to 8 panelists out of 8 panelists answered that the spreadability during use was good and the usability was good.
△ : 5 to 6 panelists out of 8 panelists answered that the spreadability during use was good and the usability was good.
× : 4 or more panelists out of 8 panelists answered that the spreadability during use was bad and the usability was not good.

### <Skin irritation>

In order to determine the skin irritation property of the cosmetic composition of the present invention, the irritation feeling to the skin was determined by eight professional panelists.

### Determination criteria for "skin irritation"

⊚ : 8 panelists out of 8 panelists answered that there was no irritation.
○ : 7 panelists out of 8 panelists answered that there was no irritation.
△ : 6 panelists out of 8 panelists answered that there was no irritation.
× : 3 panelists or more out of 8 panelists answered that there was irritation.

### <Rinsability>

To determine the rinsability of the cosmetic composition of the present invention, the rinsability was determined by eight professional panelists.

### Determination criteria for "rinsability"

○ : 7 to 8 panelists out of 8 panelists answered that it was easy to rinse after makeup removal.
△ : 5 to 6 panelists out of 8 panelists answered that it was easy to rinse after makeup removal.
× : 4 or more panelists out of 8 panelists answered that it was difficult to rinse after makeup removal.

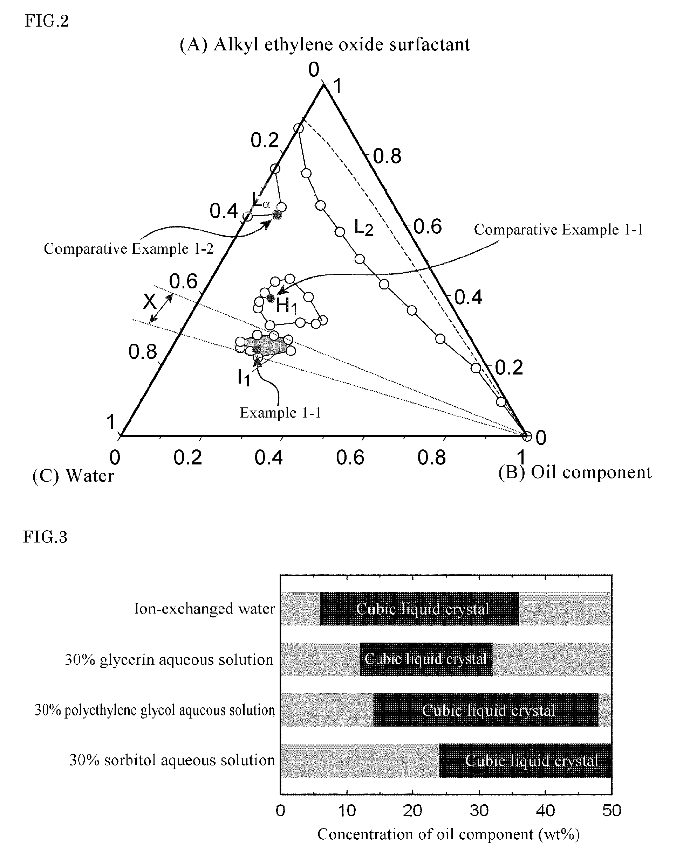

The cosmetic compositions were prepared by the formulations shown in the Table 1 below. The pseudo-three component system phase diagram (water-surfactant-oil), with the use of the components of Example 1-1, Comparative Example 1-1, and Comparative Example 1-2, is shown in Fig. 2. From the phase equilibrium diagram, Example 1-1 was found to form a discontinuous-micellar cubic liquid-crystal phase (I₁). Comparative Example 1 was found to form a hexagonal liquid crystal phase (H₁), and Comparative Example 1-2 was found to form a lamellar liquid crystal phase (L_{α}).

Furthermore, the compositions were evaluated based on the above-described evaluation criteria. The results are also shown in Table 1.

**TABLE 1**

| Component | | Example 1-1 | Comparative Example 1-1 | Comparative Example 1-2 |
|---|---|---|---|---|
| (A) | POE (10) isostearyl ther (HLB:11) *1 | 23 | 40 | 62 |
| (B) | Liquid paraffin | 16 | 16 | 6.4 |
| | Cetyl 2-ethylhexanoate | 4 | 4 | 1.6 |
| (C) | lon-exchanged water | 57 | 40 | 30 |
| Association state | | I₁ | H₁ | L_{α} |
| Cleansing effect | | ⊚ | ○ | ○ |
| Absence of dripping | | ○ | ○ | ○ |
| Good spreadability | | ○ | Δ | ○ |
| Skin irritation | | ⊚ | ○ | × |
| Rinsability | | ○ | ○ | ○ |

| | | | | |
|---|---|---|---|---|
| *1: EMALEX 1810 (manufactured by Nihon Emulsion Co., Ltd.) | | | | |

Preparation method: (A) POE (10) isostearyl ether was dissolved by heating and mixed, by stirring, with (C) water and (B) an oil component. Then, it was allowed to cool to room temperature, and the appearance of the prepared composition was observed at room temperature.

In the examples shown in Table 1 and Fig. 2, when the ratio of (A) alkyl ethylene oxide surfactant and (C) water is in the X region, a discontinuous-micellar cubic liquid-crystal phase (I₁) region is reached by the addition of the oil component (18 to 32%). That is, in the case of the component constitution of Fig. 2, it is understood that the range of POE (10) isostearyl ether:water =28:72 to 38:62 is the X region.

The mass ratio of components (A) and (C) (namely, X region), at which the I₁ region is reached by the addition of the oil component, varies depending upon the used surfactant and the constitution of other components. The present inventors have investigated the ratio in further detail. As a result, the mass ratio (A):(C) was 20:80 to 70:30 and preferably 20:80 to 45:55.

From the results of Table 1, it was found that the cosmetic composition of Example 1, wherein a surfactant having an HLB of 11 was contained and 20 mass % of oil was contained, showed a discontinuous cubic liquid-crystal phase and that the cosmetic composition was an excellent cleansing composition, which was not drippy, had a high cleansing effect, and could be cleanly rinsed off. However, in Comparative Example 1-1, wherein a hexagonal liquid crystal phase was shown, the spreadability during use was not good. In Comparative Example 1-2, wherein a lamellar liquid crystal phase was shown, the content of the surfactant was high, a slight skin irritation was observed, and the spreadability during use was slightly poor.

Subsequently, the present inventors investigated the relationship with the optimum HLB of the (A) alkyl ethylene oxide surfactant.

**TABLE 2**

| Component | | Production Example 1-1 | Production Example 1-2 | Production Example 1-3 | Production Example 1-4 | Production Example 1-5 | Production Example 1-6 |
|---|---|---|---|---|---|---|---|
| (A) | POE (5) cetyl ether *2 | 20 | - | - | - | - | - |
| | POE (5) lauryl ether *3 | - | 20 | - | - | - | - |
| | POE (7) lauryl ether *4 | - | - | 10 | - | - | - |
| | POE (9) lauryl ether *5 | - | - | 10 | - | - | - |
| | POE (12) lauryl ether *6 | - | - | - | 20 | - | - |
| | POE (15) lauryl ether *7 | - | - | - | - | 20 | - |
| | POE (2) cetyl ether *8 | - | - | - | - | - | 20 |
| (B) | Isododecane | 30 | 30 | 10 | 10 | 2 | 20 |
| (C) | Ion-exchanged water | Balance | Balance | Balance | Balance | Balance | Balance |
| HLB of Component (A) | | 8 | 10 | 11.5 | 13 | 14 | 5 |
| Association state | | I₁ | I₁ | I₁ | I₁ | I₁ | - |
| Cleansing effect | | ⊚ | ⊚ | ○ | ○ | △ | △ |
| Absence of dripping | | ○ | ○ | ○ | ○ | ○ | ○ |
| Good spreadability | | ○ | ○ | ○ | ○ | △ | △ |
| Skin irritation | | ⊚ | ⊚ | ⊚ | ⊚ | ○ | ○ |
| Rinsability | | ○ | ○ | ○ | ○ | ○ | × |

| | | | | | | | |
|---|---|---|---|---|---|---|---|
| *2: EMALEX 105 (manufactured by Nihon Emulsion Co., Ltd.) *3: EMALEX 705 (manufactured by Nihon Emulsion Co., Ltd.) *4: EMALEX 707 (manufactured by Nihon Emulsion Co., Ltd.) *5: EMALEX 709 (manufactured by Nihon Emulsion Co., Ltd.) *6: EMALEX 712 (manufactured by Nihon Emulsion Co., Ltd.) *7: EMALEX 715 (manufactured by Nihon Emulsion Co., Ltd.) *8: EMALEX 102 (manufactured by Nihon Emulsion Co., Ltd.) | | | | | | | |

Preparation method: Each (A) alkyl ethylene oxide surfactant was dissolved by heating, and then the component (C) water and the component (B) an oil component were mixed and stirred. Then, the appearance of the prepared composition was observed.

From the results of Table 2, the cosmetic compositions of Production Examples 1-1 to 1-4, wherein an (A) alkyl ethylene oxide surfactant having an HLB of 8 to 13 was contained and 10 mass % or more of isododecane was contained as the (B) oil component, was found to be an excellent cleansing composition, which showed a discontinuous cubic liquid-crystal phase (one phase), was not drippy, had a high cleansing effect, and could be cleanly rinsed off. However, in Production Example 1-5, wherein a surfactant having an HLB of 14 is contained, only 2 mass % of oil could be contained even when a discontinuous-micellar cubic liquid-crystal phase was formed, thus a satisfactory cleansing effect could not be not obtained. In Production Example 1-6, wherein a surfactant having an HLB of 5 was contained, a discontinuous-micellar cubic liquid-crystal phase could not be obtained, and the satisfactory cleansing effect, usability, and rinsability could not be obtained.

Accordingly, in the present invention, the HLB of the (A) alkyl ethylene oxide surfactant is preferably 8 to 13 and more preferably 8 to 10.

Subsequently, the present inventors investigated the blending quantity of the oil component (B) that can be blended in the cosmetic composition of the present invention.

**TABLE 3**

| Component | | Production Example 2-1 | Production Example 2-2 | Production Example 2-3 | Production Example 2-4 | Production Example 2-5 | Production Example 2-6 |
|---|---|---|---|---|---|---|---|
| (A) | POE (10) isostearyl ether (HLB: 11) *1 | 27 | 25 | 25 | 35 | 66.5 | 20 |
| (B) | Liquid paraffin | 14.4 | 20 | 24 | 16.6 | 4 | 62 |
| | Cetyl 2-ethylhexanoate | 3.6 | 5 | 6 | 4.2 | 1 | 15.5 |
| (C) | Ion-exchanged water | Balance | Balance | Balance | Balance | Balance | Balance |
| Association state | | I₁ | I₁ | I₁ | H₁ | L_{α} | L2 |
| Cleansing effect | | ⊚ | ⊚ | ⊚ | ○ | △ | ⊚ |
| Absence of dripping | | ○ | ○ | ○ | ○ | ○ | × |
| Good spreadability | | ○ | ○ | ○ | △ | ○ | ○ |
| Skin irritation | | ⊚ | ⊚ | ⊚ | ○ | × | ⊚ |
| Rinsability | | ○ | ○ | ○ | ○ | ○ | × |

| | | | | | | | |
|---|---|---|---|---|---|---|---|
| *1: EMALEX 1810 (manufactured by Nihon Emulsion Co., Ltd.) | | | | | | | |

Preparation method: (A) POE (10) isostearyl ether and the component (C) water were mixed. The dissolution by heating to about 70 °C was carried out. Then, the component (B) oil component was gradually added with stirring and mixed by stirring. Then, the appearance of the prepared composition was observed.

From the results of Table 3, the cosmetic compositions of Production Examples 2-1 to 2-3, wherein an alkyl ethylene oxide surfactant having an HLB of 8 to 13 was contained and 10 mass % or more of oil was contained, was found to be an excellent cleansing composition, which showed a discontinuous cubic liquid-crystal phase (one phase), was not drippy, had a high cleansing effect, and could be cleanly rinsed off. However, in Production Example 2-4, wherein a hexagonal liquid crystal phase was shown, the spreadability during use was not good. In Production Example 2-5, wherein only 5 mass % of oil was contained, a lamellar liquid crystal phase was shown, and skin irritation was observed because of the high content of surfactant. In Production Example 2-6, wherein excess oil was contained, a liquid crystal structure was not taken and a reverse-micellar oil solution phase was shown. As a result, the viscosity was low and it was drippy, and the satisfactory rinsability could not be achieved.

In addition, the following investigation was carried out concerning the blending of the (D) polyhydric alcohol.

At first, to the mixture wherein a surfactant (POE (10) cetyl ether) and ion-exchanged water or a polyhydric alcohol aqueous solution (30% glycerin aqueous solution, 30% polyethylene glycol aqueous solution, or 30% sorbitol aqueous solution) were mixed in a mass ratio of 38:62 (surfactant:aqueous solution), an oil component (isododecane) was gradually added. The concentration of the oil component was gradually increased, and the concentration range wherein the composition phase state becomes a discontinuous-micellar cubic liquid-crystal phase (I₁ phase) was investigated. The results are shown in Fig. 3.

As shown in Fig. 3, in the sample wherein a glycerin aqueous solution was used, the upper concentration limit of the oil component at which the composition can form an I₁ phase (region shown by "cubic liquid crystal" in Fig. 3) was slightly lower than the case wherein ion-exchanged water was used. On the other hand, in the sample wherein a polyethylene glycol aqueous solution or sorbitol aqueous solution was used, the upper concentration limit of the oil component drastically increased by the blending of the polyhydric alcohol compared with the non-blending case (ion-exchanged water).

Thus, it is clear in the present invention that a cosmetic composition with a discontinuous-micellar cubic liquid-crystal phase retaining a larger amount of (B) an oil component can be obtained by blending a polyhydric alcohol, in particular, polyethylene glycol or sorbitol.

Subsequently, the actual use tests were carried out for each production example (makeup remover) prepared by the formulations shown in the Table 4 below, and each was evaluated according to the above-described evaluation criteria. The blending quantity of an oil component in each production example was set based on the concentration range shown in Fig. 3. The results are shown in Table 4.

**TABLE 4**

| | Production Example 3-1 | Production Example 3-2 | Production Example 3-3 | Production Example 3-4 |
|---|---|---|---|---|
| POE (10) cetyl ether | 28.88 | 27.36 | 24.32 | 22.80 |
| Glycerin | | 13.39 | | |
| Polyethylene glycol | | | 11.90 | |
| Sorbitol | | | | 11.16 |
| Isododecane | 16.80 | 19.60 | 25.20 | 28.00 |
| Cetyl2-ethylhexanoate | 7.20 | 8.40 | 10.80 | 12.00 |
| Ion-exchanged water | Balance | Balance | Balance | Balance |
| HLB of surfactant | 11 | 11 | 11 | 11 |
| Association state | I₁ | I₁ | I₁ | I₁ |
| Cleansing effect | ○ | ○ | ⊚ | ⊚ |
| Absence of dripping | ○ | ○ | ○ | ○ |
| Good spreadability | ○ | ⊚ | ⊚ | ⊚ |
| Skin irritation | ⊚ | ⊚ | ⊚ | ⊚ |
| Rinsability | ○ | ○ | ○ | ○ |

As shown in Table 4, all the compositions formed a discontinuous-micellar cubic liquid-crystal phase. However, Production Example 3-2, wherein glycerin was blended, had more excellent composition spreadability compared with Production Example 3-1, wherein a polyhydric alcohol was not blended.

In Production Examples 4-3 and 4-4, wherein polyethylene glycol or sorbitol was blended, the cleansing effect was extremely high in addition to the improved spreadability. As shown in Fig. 3, this is considered because the concentration of the oil component was increased by both components.

Thus, in the present invention, the cosmetic compositions with much better spreadability can be obtained by blending a polyhydric alcohol as the component (D). In particular, by using polyethylene glycol or sorbitol as the polyhydric alcohol, a larger amount of the oil component can be blended; as a result, the cosmetic compositions with a high cleansing effect can be obtained.

Here are examples of the present invention.

### EXAMPLE 1-2 Makeup remover

| (Component) | (% by mass) |
|---|---|
| (1) POE (10) isostearyl ether | 20 |
| (EMALEX 1810, manufactured by Nihon Emulsion Co., Ltd.) | |
| (2) POE (15) isostearyl ether | 6 |
| (EMALEX 1815, manufactured by Nihon Emulsion Co., Ltd.) | |
| (3) Liquid paraffin | 15 |
| (4) Isododecane | 5 |
| (5) Cetyl 2-ethylhexanoate | 6 |
| (6) Antiseptic agent | Appropriate amount |
| (7) Fragrance | Appropriate amount |
| (8) Ion-exchanged water | Balance |

### (Preparation method)

(1), (2), (6) and (8) were weighed and dissolved by heating to about 70 °C. Then (3)-(5) and (7) were gradually added to there with stirring, and stirred and mixed. And then the product was confirmed to have a discontinuous-micellar cubic liquid-crystal phase.

### EXAMPLE 1-3 Makeup remover

| (Constituent) | (% by mass) |
|---|---|
| (1) POE (15) isoceryl ether | 20 |
| (EMALEX 1615, manufactured by Nihon Emulsion Co., Ltd.) | |
| (2) Ethanol | 5 |
| (3) Isododecane | 8 |
| (4) Decamethylcyclopentasiloxane | 2 |
| (5) Antiseptic agent | Appropriate amount |
| (6) Fragrance | Appropriate amount |
| (7) Ion-exchanged water | Balance |

### (Preparation method)

(1), (2), (5) and (7) were weighed and dissolved by heating to about 70 °C. Then (3), (4) and (6) were gradually added to there with stirring, and stirred and mixed. And then the product was confirmed to have a discontinuous-micellar cubic liquid-crystal phase.

### EXAMPLE 1-4 Makeup remover

| (Component) | (% by mass) |
|---|---|
| (1) POE (5) ceryl ether | 13 |
| (EMALEX 105, manufactured by Nihon Emulsion Co., Ltd.) | |
| (2) POE (7) cetyl ether | 12 |
| (EMALEX 107, manufactured by Nihon Emulsion Co., Ltd.) | |
| (3) Isododecane | 20 |
| (4) Isohexadecane | 10 |
| (5) Glyceryl tri(2-ethylhexanoate) | 2 |
| (6) Antiseptic agent | Appropriate amount |
| (7) Fragrance | Appropriate amount |
| (8) Ion-exchanged water | Balance |

### (Preparation method)

(1), (2), (6) and (8) were weighed and dissolved by heating to about 70 °C. Then (3)-(5) and (7) were gradually added to there with stirring, and stirred and mixed. And then the product was confirmed to have a discontinuous-micellar cubic liquid-crystal phase.

### EXAMPLE 1-5 Hair coloring preparation

| (Component) | (% by mass) |
|---|---|
| (1) POE (10) ceryl ether | 20 |
| (EMALEX 110, manufactured by Nihon Emulsion Co., Ltd.) | |
| (2) Isododecane | 20 |
| (3) Isohexadecane | 5 |
| (4) Strong ammonia water | 8.6 |
| (5) Ethanolamine | 2.4 |
| (6) Hydrosulfite sodium (reagent) | 0.1 |
| (7) Ascorbic acid | 0.3 |
| (8) Cationic silk protein | 0.1 |
| (9) Disodium edetate | 0.2 |
| (10) Resorcin | 1.0 |
| (11) Purified water | Balance |
| (12) Fragrance | Appropriate amount |

### (Preparation method)

(1)-(3) and (11) were weighed, dissolved by heating to about 70 °C, then stirred and mixed. The rest of constituents were gradually added to there with stirring and cooling, and mixed. And then the product was confirmed to have a discontinuous-micellar cubic liquid-crystal phase.

### EXAMPLE 1-6 Hair conditioner

| (Component) | (% by mass) |
|---|---|
| (1) POE (5) cetyl ether | 15 |
| (EMALEX 105, manufactured by Nihon Emulsion Co., Ltd.) | |
| (2) POE (7) cetyl ether | 15 |
| (EMALEX 107, manufactured by Nihon Emulsion Co., Ltd.) | |
| (3) Liquid paraffin | 20 |
| (4) Cetyl 2-ethylhexanoate | 8 |
| (5) Squalane | 0.1 |
| (6) Pyroglutamic acid glyceryl oleate | 0.05 |
| (7) Stearyl trimethyl ammonium chloride (80%) | 1.15 |
| (8) Hydrolyzed keratin solution | 0.1 |
| (9) N-[2-hydroxy-3-(trimethylammonio)propyl] hydrolyzed collagen chloride | 0.5 |
| (10) P-hydroxybenzoate ester | Appropriate amount |
| (11) Hydroxyethyl cellulose | 1.0 |
| (12) Fragrance | Appropriate amount |
| (13) Ion-exchanged water | Balance |

### (Preparation method)

(1)-(5) and (13) were weighed, dissolved by heating to about 70 °C, then stirred and mixed. The rest of constituents were gradually added to there with stirring and cooling, and mixed. And then the product was confirmed to have a discontinuous-micellar cubic liquid-crystal phase.

### EXAMPLE 1-7 Makeup remover

| (Component) | (% by mass) |
|---|---|
| (1) POE (10) isostearyl ether | 18.38 |
| (EMALEX 1810, manufactured by Nihon Emulsion Co., Ltd.) | |
| (2) POE (10) cetyl ether | 6.13 |
| (EMALEX 110, manufactured by Nihon Emulsion Co., Ltd.) | |
| (3) Ethanol | 0.50 |
| (4) Polyethylene glycol | 8.19 |
| (5) 1,3-butylene glycol | 5.46 |
| (6) Isododecane | 21.00 |
| (7) Polymethylsiloxane | 3.00 |
| (8) Octyl palmitate | 6.00 |
| (9) Antiseptic agent | Appropriate amount |
| (10) Fragrance | Appropriate amount |
| (11) Ion-exchanged water | Balance |

### (Preparation method)

(1)-(5), (9) and (11) were weighed and dissolved by heating to about 70 °C. Then (6)-(8) and (10) were gradually added to there with stirring, and stirred and mixed. And then the product was confirmed to have a discontinuous-micellar cubic liquid-crystal phase.

Secondly, the present inventors investigated the (O/I₁) composition, wherein the discontinuous-micellar cubic liquid-crystal phase is the outer phase and an oil component different from the oil component in the micelle of the liquid crystal is contained as the inner phase of the system. In the present examples, the same evaluation methods were adopted as those for the above-described example.

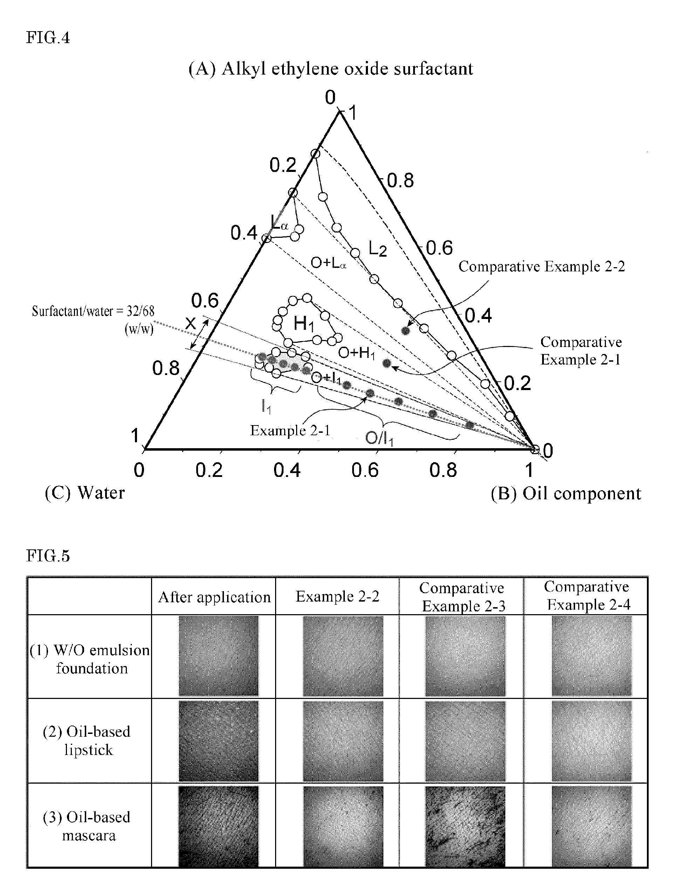

The cosmetic compositions were prepared by the formulations shown in the Table 1 below. The pseudo-three component system phase diagram (water-surfactant-oil), with the use of the components of Example 1, Comparative Examples 1, and Comparative Example 2, is shown in Fig. 4. From all of Example 2-1, Comparative Example 2-1, and Comparative Example 2-2, wherein 50% of oil is contained, an oil-in-liquid crystal type emulsion composition was obtained. However, it was found from the phase equilibrium diagram in Fig. 4 that the outer phase of the emulsion composition of Example 2-1 was a discontinuous-micellar cubic liquid-crystal phase, the outer phase of emulsion composition of Comparative Example 1 was a hexagonal liquid crystal phase, and the outer phase of the emulsion composition of Comparative Example 2-2 was a lamellar liquid crystal phase.

Furthermore, the cosmetic compositions were evaluated based on the above-described evaluation criteria. The results are also shown in Table 5.

**TABLE 5**

| Component | | Example 2-1 | Comparative Example 2-1 | Comparative Example 2-2 |
|---|---|---|---|---|
| (A) | POE (10) isostearyl ether (HLB: 11) *1 | 16 | 27 | 35 |
| (B) | Liquid paraffin | 40 | 40 | 40 |
| | Cetyl 2-ethylhexanoate | 10 | 10 | 10 |
| (C) | lon-exchanged water | Balance | Balance | Balance |
| Association state of outer phase | | I₁ | H₁ | L_{α} |
| Oil concentration in cubic liquid-crystal phase (B₁) | | 28 | 32 | |
| Cleansing effect | | ⊚ | ⊚ | △ |
| Absence of dripping | | ○ | ○ | ○ |
| Good spreadability | | ○ | △ | △ |
| Skin irritation | | ⊚ | ○ | △ |
| Rinsability | | ○ | ○ | △ |
| Temporal stability | | ○ | Δ | × |

| | | | | |
|---|---|---|---|---|
| *1: EMALEX 1810 (manufactured by Nihon Emulsion Co., Ltd.) | | | | |

Preparation method: (A) POE (10) isostearyl ether and the component (C) water were mixed. The dissolution by heating to about 70 °C was carried out. Then, the component (B) oil component was gradually added with stirring and mixed by stirring. Then, the appearance of the prepared composition was observed.

In the examples shown in Table 5 and Fig. 4, when the ratio of (A) alkyl ethylene oxide surfactant and (C) water is in the X region, a discontinuous-micellar cubic liquid-crystal phase (I₁) region is reached by the addition of the oil component (18 to 32%). That is, in the case of the component constitution of Fig. 4, it is understood that the range of POE (10) isostearyl ether:water =28:72 to 38:62 is the X region.

The mass ratio of components (A) and (C) (namely, X region), at which the I₁ region is reached by the addition of the oil component, varies depending upon the used surfactant and the constitution of other components. The present inventors have investigated the ratio in further detail. As a result, the mass ratio (A):(C) was 20:80 to 70:30 and preferably 20:80 to 45:55.

From the results of Table 5, it was found that the cosmetic composition of Example 2-1, wherein a surfactant having an HLB of 11 was contained and 50 mass % of oil was contained, showed a discontinuous cubic liquid-crystal phase for the outer phase and that the cosmetic composition was an excellent one, which was not drippy, had a high cleansing effect, could be cleanly rinsed off, and had a high stability. However, in Comparative Example 2-1, wherein the outer phase showed a hexagonal liquid crystal phase, the spreadability during use was not good and there was a problem in stability. In Comparative Example 2-2, wherein the outer phase showed a lamellar liquid crystal phase, the content of the surfactant was high, some skin irritation was observed, the spreadability during use was not good, and the stability was low.

Subsequently, the present inventors investigated the relationship with the optimum HLB of the (A) alkyl ethylene oxide surfactant.

**TABLE 6**

| Component | | Production Example 1-1 | Production Example1-2 | Production Example 1-3 | Production Example 1-4 | Production Example 1-5 | Production Example 1-6 |
|---|---|---|---|---|---|---|---|
| (A) | POE (5) cetyl ether *2 | 19 | - | - | - | - | - |
| | POE (5) lauryl ether *3 | - | 19 | - | - | - | - |
| | POE (7) lauryl ether *4 | - | - | 10 | - | - | - |
| | POE (9) lauryl ether *5 | - | - | 10 | - | - | - |
| | POE (12) lauryl ether *6 | - | - | - | 20 | - | - |
| | POE (15) lauryl ether *7 | - | - | - | - | 38 | - |
| | POE (2) cetyl ether *8 | - | - | - | - | - | 25 |
| (B) | Isododecane | 50 | 50 | 50 | 50 | 50 | 50 |
| (C) | lon-exchanged water | Balance | Balance | Balance | Balance | Balance | Balance |
| HLB of component (A) | | 8 | 10 | 11.5 | 13 | 14 | 5 |
| Oil concentration in cubic liquid-crystal phase (B₁) | | 26.9 | 12.5 | 5.6 | 3.2 | 2.1 | - |
| Association state of outer phase | | I₁ | I₁ | I₁ | I₁ | I₁ | - |
| Cleansing effect | | ⊚ | ⊚ | ○ | ○ | ○ | △ |
| Absence of dripping | | ○ | ○ | ○ | ○ | ○ | ○ |
| Good spreadability | | ○ | ○ | ○ | △ | △ | △ |
| Skin irritation | | ⊚ | ⊚ | ⊚ | ⊚ | ○ | ○ |
| Rinsability | | ○ | ○ | ○ | ○ | ○ | × |
| Temporal stability | | ○ | ○ | ○ | ○ | △ | × |

| | | | | | | | |
|---|---|---|---|---|---|---|---|
| *2: EMALEX 105 (manufactured by Nihon Emulsion Co., Ltd.) *3: EMALEX 705 (manufactured by Nihon Emulsion Co., Ltd.) *4: EMALEX 707 (manufactured by Nihon Emulsion Co., Ltd.) *5: EMALEX 709 (manufactured by Nihon Emulsion Co., Ltd.) *6: EMALEX 712 (manufactured by Nihon Emulsion Co., Ltd.) *7: EMALEX 715 (manufactured by Nihon Emulsion Co., Ltd.) *8: EMALEX 102 (manufactured by Nihon Emulsion Co., Ltd.) | | | | | | | |

Preparation method: Each (A) alkyl ethylene oxide surfactant and the component (C) water were mixed. The dissolution by heating to about 70 °C was carried out. Then, the component (B) oil component was gradually added with stirring and mixed by stirring. Then, the appearance of the prepared composition was observed.

From the results of Table 6, it was found that the cosmetic compositions of Production Examples 1-1 to 1-4, wherein an alkyl ethylene oxide surfactant having an HLB of 8 to 13 and 50 mass % of isododecane were contained, were an oil-in-micellar cubic liquid-crystal phase (O/I₁)-type emulsion composition, wherein the outer phase was a discontinuous cubic liquid-crystal phase, and that they were excellent cosmetic compositions which were not drippy, had a high cleansing effect, and could be cleanly rinsed off. In particular, in the cases of Production Examples 1-1 to 1-3, wherein a surfactant having an HLB of 8 to 10 was used, 5 to 30 mass % of oil could be retained inside the micelle (B1). Thus, a significant improvement in the cleansing effect was observed compared with the case of Production Example 1-4, wherein an alkyl ethylene oxide surfactant having an HLB of 13 was used.

On the other hand, in Production Example 1-5, wherein a surfactant having an HLB of 14 was contained, an emulsion having a discontinuous-micellar cubic liquid-crystal phase as the outer phase could be obtained. However, the amount of oil retainable in the micelle (B1) was small; thus the spreadability was not good and there was some problem in the stability over time. In Production Example 1-6, wherein a surfactant having an HLB of 5 was contained, a discontinuous-micellar cubic liquid-crystal phase was not formed, and a satisfactory cleansing effect, usability, rinsability, and stability could not be obtained.

Accordingly, in the present invention, the HLB of the (A) alkyl ethylene oxide surfactant is preferably 8 to 13 and more preferably 8 to 10.

Subsequently, the present inventors investigated the blending quantity of the (B) oil component that can be blended in the cosmetic composition of the present invention.

**TABLE 7**

| Component | | Production Example 2-1 | Production Example 2-2 | Production Example 2-3 | Production Example 2-4 | Production Example 2-5 |
|---|---|---|---|---|---|---|
| | (A) POE (7) lauryl ether *4 | 18.5 | 16 | 12 | 8.0 | 4 |
| | POE (9) lauryl ether *5 | 18.5 | 16 | 12 | 8.0 | 4 |
| (B) | Isohexadecane | 6.4 | 16 | 32.0 | 48 | 64 |
| | Cetyl2-ethylhexanoate | 1.6 | 4.0 | 8.0 | 12 | 16 |
| (C) | Ion-exchanged water | Balance | Balance | Balance | Balance | Balance |
| Total amount of oil (B) | | 8 | 20 | 40 | 60 | 80 |
| HLB of surfactant (A) | | 11.5 | 11.5 | 11.5 | 11.5 | 11.5 |
| Association state of outer phase | | I₁ | I₁ | I₁ | I₁ | I₁ |
| Cleansing effect | | △ | ○ | ⊚ | ⊚ | ⊚ |
| Absence of dripping | | ○ | ○ | ○ | ○ | ○ |
| Good spreadability | | △ | ○ | ○ | ○ | ○ |
| Skin irritation | | ⊚ | ⊚ | ⊚ | ⊚ | ⊚ |
| Rinsability | | ○ | ○ | ○ | ○ | ○ |
| Temporal stability | | ○ | ○ | ○ | ○ | △ |

| | | | | | | |
|---|---|---|---|---|---|---|
| *4: EMALEX 707 (manufactured by Nihon Emulsion Co., Ltd.) *5: EMALEX 709 (manufactured by Nihon Emulsion Co., Ltd.) | | | | | | |

Preparation method: (A) Alkyl ethylene oxide surfactant and the component (C) water were mixed. The dissolution by heating to about 70 °C was carried out. Then, the component (B) oil component was gradually added with stirring and mixed by stirring. Thus, an oil-in-micellar cubic liquid-crystal phase (O/I₁)-type emulsion composition, wherein the outer phase is a discontinuous cubic liquid-crystal phase, was obtained.

From the results of Table 7, Production Examples 2-2 to 2-5, wherein 40 mass % or more of oil was contained, were excellent ones, which were not drippy, had a high cleansing effect, and could be cleanly rinsed off.

Accordingly, in the present invention, the total amount of oil in the cosmetic composition is 20 to 80 mass % and preferably 40 to 80 mass %.

Next, the present inventors show the compositions for Example 2-2, which is the cosmetic composition of the present invention, Comparative Example 2-3, which is a conventional water-based cleansing gel makeup remover, and Comparative Example 2-4, which is a conventional cleansing oil, and their evaluations in Table 8 and Fig. 5.

### <Makeup removing effect>

In order to determine the makeup removing effect of the cosmetic composition of the present invention, (1) W/O emulsion foundation, (2) oil-based lipstick, and (3) low-water-content oil-based mascara were applied on the skin, respectively, and the makeup removal was visually evaluated.

### Determination criteria for "makeup removing effect"

⊚ : Makeup dirt residue is hardly observed on the skin.
○ : Slight makeup dirt residue is observed on the skin.
△ : Some makeup dirt residue is observed on the skin.
× : Makeup dirt residue is observed on the skin.

### Example 2-2

| (Component) | (% by mass) |
|---|---|
| (1) POE (10) isostearyl ether | 10 |
| (EMALEX 1810, manufactured by Nihon Emulsion Co., Ltd.) | |
| (2) Isododecane | 50 |
| (3) Cetyl 2-ethylhexanoate | 20 |
| (4) Fragrance | Appropriate amount |
| (5) Ion-exchanged water | Balance |

### Comparative Example 2-3 Cleansing gel

| (Component) | (% by mass) |
|---|---|
| (1) Polyoxyethylene/methylpolysiloxane copolymer | 5 |
| (2) Dipropylene glycol | 3 |
| (3) 1,3-butylene glycol | 10 |
| (4) Polyethylene glycol 1500 | 1 |
| (5) Polyoxyethylene methyl glucoside | 1 |
| (6) Polyethylene glycol diisostearate | 5 |
| (7) Dipotassium glycyrrhizate | 0.1 |
| (8) Cola de caballo extract | 0.1 |
| (9) Edetate trisodium | 0.01 |
| (10) Hydroxypropyl methyl cellulose | 0.8 |
| (11) Carboxyvinyl polymer-potassium | 0.4 |
| (12) P-hydroxybenzoate ester | Appropriate amount |
| (13) Purified water | Balance |

### Comparative Example 2-4 Cleansing oil

| (Component) | (% by mass) |
|---|---|
| (1) Liquid paraffin | 49 |
| (2) Cetyl 2-ethylhexanoate | 20 |
| (3) POE (8) isostearate (HLB = 9, liquid form) | 30 |
| (4) Isostearic acid | 5 |
| (5) Purified water | 1 |
| (6) BHT | Appropriate amount |
| (7) Fragrance | Appropriate amount |

**TABLE 8**

| | Example 2-2 | Comparative Example 2-3 | Comparative Example 2-4 |
|---|---|---|---|
| (1) W/O emulsion foundation | ⊚ | ○ | ⊚ |
| (2) Oil-based lipstick | ○ | ○ | ⊚ |
| (3) Oil-based mascara | ○ | × | ○ |

From the above Table 8 and Fig. 5, it became clear that the cosmetic composition of the present invention has a very high makeup removing effect compared with the conventional water-based cleansing gel makeup remover (Comparative Example 2-2). In addition, it became clear that the cosmetic composition of the present invention has a comparable cleansing effect to the conventional cleansing oil (Comparative Example 2-4); nevertheless, the rinsability is excellent.

Furthermore, the following investigation was carried out concerning the blending of (D) polyhydric alcohol.

Actual use tests of each production example (makeup remover) prepared by the formulations shown in the below Table 9 were carried out, and each was evaluated according to the above-described evaluation criteria and the following evaluation criteria (transparency of the base). The results are shown in Table 5.

### < Transparency of composition>

The visible light transmittance (light path length: 0.5 cm) was measured at the wavelength 600 nm with a spectrophotometer (Hitachi U3510), and the composition transparency of each production example was evaluated according to the below-described criteria.
A: The transmittance is 80% or higher.
B: The transmittance is 60% or higher and less than 80%.
C: The transmittance is 30% or higher and less than 60%.
D: The transmittance is 10% or higher and less than 30%.
E : The transmittance is less than 10%.

**TABLE 9**

| | Production Example 3-1 | Production Example 3-2 | Production Example 3-3 | Production Example 3-4 | Production Example 3-5 | Production Example 3-6 |
|---|---|---|---|---|---|---|
| (A) POE (10) cetyl ether | 15.20 | 15.20 | 15.20 | 15.20 | 15.20 | 15.20 |
| (B) Isododecane | 42.00 | 42.00 | 42.00 | 42.00 | 42.00 | 42.00 |
| (B) Cetyl 2-ethylhexanoate | 18.00 | 18.00 | 18.00 | 18.00 | 18.00 | 18.00 |
| (C) lon-exchanged water | Balance | Balance | Balance | Balance | Balance | Balance |
| (D) Glycerin | | 2.48 | 4.96 | 7.44 | 9.92 | 12.40 |
| Total amount of oil (B) | 60.00 | 60.00 | 60.00 | 60.00 | 60.00 | 60.00 |
| HLB of surfactant (A) | 11 | 11 | 11 | 11 | 11 | 11 |
| Ratio of water (C):polyhidric alcohol (D) | 100 : 0 | 90 : 10 | 80 : 20 | 70 : 30 | 60 : 40 | 50 : 50 |
| Association state of outer phase | I₁ | I₁ | I₁ | I₁ | I₁ | I₁ |
| Cleansing effect | ⊚ | ⊚ | ⊚ | ⊚ | ⊚ | ⊚ |
| Absence of dripping | ○ | ○ | ○ | ○ | ○ | ○ |
| Good spreadability | ○ | ○ | ⊚ | ⊚ | ⊚ | ⊚ |
| Skin irritation | ⊚ | ⊚ | ⊚ | ⊚ | ⊚ | ⊚ |
| Rinsability | ○ | ○ | ○ | ○ | ○ | ○ |
| Transparency of composition | E | D | C | B | A | A |

Preparation method: components (A), (C) and (D) were blended and dissolved by heating to about 70 °C. Then component (B) was gradually added to there with stirring, and stirred and mixed to obtain an oil-in-micellar cubic liquid-crystal phase (O/I₁)-type emulsion composition having a discontinuous cubic liquid-crystal phase as the outer phase.

As shown in Table 9, the composition transparency increased with an increase of added glycerin (polyhydric alcohol) to the composition. Production Example 3-1, wherein no polyhydric alcohol was blended, showed a creamy appearance. However, the composition appearance of Production Examples 3-5 and 3-6 became transparent and gel-like.

In Production Examples 3-3 to 3-6, wherein the ratio of the polyhydric alcohol to water was 20% or higher, the spreadability improved compared with other examples.

Thus, in the present invention, by adding a polyhydric alcohol as the component (D), the composition appearance (transparency) can be varied while maintaining the cleansing effect and the texture in use. The appearance can be sufficiently changed by allowing the blending quantity of a polyhydric alcohol to be (C):(D) = 90:10 to 60:40. However, a cosmetic composition with better spreadability can be obtained, in particular, in the range of (C):(D)=80:20 to 50:50.

Subsequently, the actual use tests for each production example (makeup remover) prepared by the formulations shown in the below Table 10 were carried out, and each was evaluated according to the above-described evaluation criteria. The results are shown in Table 10.

**TABLE 10**

| | Production Example 4-1 | Production Example 4-2 | Production Example 4-3 | Production Example 4-4 | Production Example 4-5 | Production Example 4-6 |
|---|---|---|---|---|---|---|
| (A) POE (10) cetyl ether | 10.64 | 10.64 | 10.64 | 10.64 | 10.64 | 10.64 |
| (A) POE (10) isostearyl ether | 4.56 | 4.56 | 4.56 | 4.56 | 4.56 | 4.56 |
| (B) Isododecane | 45.00 | 45.00 | 45.00 | 45.00 | 45.00 | 45.00 |
| (B) Octyl palmitate | 15.00 | 15.00 | 15.00 | 15.00 | 15.00 | 15.00 |
| (C) lon-exchanged water | Balance | Balance | Balance | Balance | Balance | Balance |
| (D) Ethanol | 7.44 | | | | | |
| (D) 1,3-butylene glycol | | 7.44 | | | | |
| (D) Dipropylene glycol | | | 7.44 | | | |
| (D) Glycerin | | | | 7.44 | | |
| (D) Polyethylene glycol | | | | | 7.44 | |
| (D) Sorbitol | | | | | | 7.44 |
| Total amount of oil (B) | 60.00 | 60.00 | 60.00 | 60.00 | 60.00 | 60.00 |
| HLB of surfactant (A) | 11 | 11 | 11 | 11 | 11 | 11 |
| Ratio of water (C):polyhydric alcohol (D) | 70:30 | 70 : 30 | 70 : 30 | 70:30 | 70 : 30 | 70: 30 |
| Association state of outer phase | I₁ | I₁ | I₁ | I₁ | I₁ | I₁ |
| Cleansing effect | ⊚ | ⊚ | ⊚ | ⊚ | ⊚ | ⊚ |
| Absence of dripping | ○ | ○ | ○ | ○ | ○ | ○ |
| Good spreadability | ○ | ⊚ | ⊚ | ⊚ | ⊚ | ⊚ |
| Skin irritation | ⊚ | ⊚ | ⊚ | ⊚ | ⊚ | ⊚ |
| Rinsability | ○ | ○ | ○ | ○ | ○ | ○ |
| Transparency of composition | D | B | B | B | B | B |

Preparation method: components (A), (C) and (D) were blended and dissolved by heating to about 70 °C. Then component (B) was gradually added to there with stirring, and stirred and mixed to obtain an oil-in-micellar cubic liquid-crystal phase (O/I₁)-type emulsion composition having a discontinuous cubic liquid-crystal phase as the outer phase.

As shown in Table 10, the transparency of a composition wherein a polyhydric alcohol was blended, regardless of its kind, increased and became transparent to translucent and gel-like. In addition, the improvement in spreadability was observed in all cases wherein a polyhydric alcohol was blended. On the other hand, in Production Example 4-1, wherein a monohydric alcohol (ethanol) was blended, a change in transparency was hardly observed by the addition of the same amount, and there was also no change in the texture in use compared with other production examples.

Thus, in the present invention, polyhydric alcohol, in particular, 1,3-butylene glycol, dipropylene glycol, glycerin, polyethylene glycol, or sorbitol can be preferably blended.

Here are examples of the present invention.

### Example 2-3 Makeup remover

| (Component) | (% by mass) |
|---|---|
| (1) POE (10) isostearyl ether | 12 |
| (EMALEX 1810, manufactured by Nihon Emulsion Co., Ltd.) | |
| (2) POE (15) isostearyl ether | 3 |
| (EMALEX 1815, manufactured by Nihon Emulsion Co., Ltd.) | |
| (3) Liquid paraffin | 20 |
| (4) Isododecane | 10 |
| (5) Cetyl 2-ethylhexanoate | 12 |
| (6) Antiseptic agent | Appropriate amount |
| (7) Fragrance | Appropriate amount |
| (8) Ion-exchanged water | Balance |

### (Preparation method)

(1), (2), (6) and (8) were weighed and dissolved by heating to about 70°C. Then (3)-(5) and (7) were gradually added to there with stirring, and stirred and mixed. And then the product was confirmed to have a discontinuous-micellar cubic liquid-crystal phase as the outer phase.

### Example 2-4 Makeup remover

| (Component) | (% by mass) |
|---|---|
| (1) POE (15) isocetyl ether | 12 |
| (EMALEX 1615, manufactured by Nihon Emulsion Co., Ltd.) | |
| (2) Ethanol | 2 |
| (3) Isododecane | 55 |
| (4) Decamethylcyclopentasiloxane | 5 |
| (5) Antiseptic agent | Appropriate amount |
| (6) Fragrance | Appropriate amount |
| (7) Ion-exchanged water | Balance |

### (Preparation method)

(1), (2), (5) and (7) were weighed and dissolved by heating to about 70 °C. Then (3), (4) and (6) were gradually added to there with stirring, and stirred and mixed. And then the product was confirmed to have a discontinuous-micellar cubic liquid-crystal phase as the outer phase.

### Example 2-5 Makeup remover

| (Component) | (% by mass) |
|---|---|
| (1) POE (5) lauryl ether | 10 |
| (EMALEX 705, manufactured by Nihon Emulsion Co., Ltd.) | |
| (2) POE (7) lauryl ether | 0.5 |
| (EMALEX 707, manufactured by Nihon Emulsion Co., Ltd.) | |
| (3) Isododecane | 50 |
| (4) Isohexadecane | 20 |
| (5) Glyceryl tri(2-ethylhexanoate) | 2 |
| (6) Antiseptic agent | Appropriate amount |
| (7) Fragrance | Appropriate amount |
| (8) Ion-exchanged water | Balance |

### (Preparation method)

(1), (2), (6) and (8) were weighed and dissolved by heating to about 70°C. Then (3)-(5) and (7) were gradually added to there with stirring, and stirred and mixed. And then the product was confirmed to have a discontinuous-micellar cubic liquid-crystal phase as the outer phase.

### Example 2-6 Hair conditioner

| (Component) | (% by mass) |
|---|---|
| (1) POE (10) cetyl ether | 15 |
| (EMALEX 110, manufactured by Nihon Emulsion Co., Ltd.) | |
| (2) Isododecane | 40 |
| (3) Ethyl 2-ethylhexanoate | 10 |
| (4) Dimethylpolysiloxane | 2 |
| (5) Stearyl alcohol | 2 |
| (6) Behenyl alcohol | 2 |
| (7) Glycerin | 1.5 |
| (8) Octyl palmitate | 1 |
| (9) Polyoxyethylene stearyl ether | 0.2 |
| (10) Citric acid | 0.05 |
| (11) P-hydroxybenzoate ester | Appropriate amount |
| (12) Phenoxyethanol | Appropriate amount |
| (13) Hydroxyethyl cellulose | 0.1 |
| (14) Stearyl trimethyl ammonium chloride | 1 |
| (15) Methylpolysiloxane with high polymerization degree | 1.5 |
| (16) Purified water | Balanece |
| (17) Fragrance | Appropriate amount |

### Example 2-7 Cream

| (Component) | (% by mass) |
|---|---|
| (1) POE (10) isostearyl ether | 12 |
| (EMALEX 1810, manufactured by Nihon Emulsion Co., Ltd.) | |
| (2) Liquid paraffin | 50 |
| (3) Cetyl 2-ethylhexanoate | 10 |
| (4) Vaseline | 1 |
| (5) Dimethylpolysiloxane | 1 |
| (6) Glycerin | 8 |
| (7) Dipropylene glycol | 5 |
| (8) Macadamia nut oil | 2 |
| (9) Hardened oil | 3 |
| (10) Squalane | 6 |
| (11) Cholesteryl hydroxystearate | 0.5 |
| (12) Polyoxyethylene hardened castor oil | 0.5 |
| (13) Sodium hexametaphosphate | 0.05 |
| (14) Trimethylglycine | 2 |
| (15) L-ascorbic acid alpha-tocopherol phosphoric acid diester potassium salt | 2 |
| (16) Tocopherol acetate | 0.1 |
| (17) Rubus suavissimus extract | 0.1 |
| (18) Paraben | Appropriate amount |
| (19) Edetate trisodium | 0.05 |
| (20) 4-t-butyl-4'-methoxy dibenzoylmethane | 0.05 |
| (21) Glyceryl mono-2-ethylhexanoyl-diparamethoxy cinnamate | 0.05 |
| (22) Color material | Appropriate amount |
| (23) Purified water | Balance |

### Example 2-8 Hair coloring preparation

| (Component) | (% by mass) |
|---|---|
| (1) POE (12) oleyl ether | 20 |
| (EMALEX 512, manufactured by Nihon Emulsion Co., Ltd.) | |
| (2) Liquid paraffin | 28 |
| (3) Isopropyl palmitate | 12 |
| (4) Dimethylpolysiloxane | 0.5 |
| (5) Propylene glycol | 5 |
| (6) Stearic acid | 3 |
| (7) Oleic acid | 4 |
| (8) Strong ammonia water | 6.6 |
| (9) Ethanolamine | 1.4 |
| (10) Ascorbic acid | 0.6 |
| (11) Cationic silk protein | 0.1 |
| (12) Disodium edetate | 0.2 |
| (13) Resorcin | 0.1 |
| (14) Purified water | Balance |
| (15) Fragrance | Appropriate amount |

### Example 2-9 Cleansing gel

| (Component) | (% by mass) |
|---|---|
| (1) POE (10) isostearyl ether | 7.88 |
| (EMALEX 1810, manufactured by Nihon Emulsion Co., Ltd.) | |
| (2) POE (10) cetyl ether | 2.63 |
| (EMALEX 110, manufactured by Nihon Emulsion Co., Ltd.) | |
| (3) Glycerin | 1.46 |
| (4) Polyethylene glycol | 0.50 |
| (5) 1,3-butylene glycol | 4.39 |
| (6) Isododecane | 49.00 |
| (7) Polymethylsiloxane | 7.00 |
| (8) Cetyl 2-ethylhexanoate | 14.00 |
| (9) Antiseptic agent | Appropriate amount |
| (10) Fragrance | Appropriate amount |
| (11) Purified water | Balance |

### (Preparation method)

(1)-(5), (9) and (11) were weighed and dissolved by heating to about 70 °C. Then (6)-(8) and (10) were gradually added to there with stirring, and stirred and mixed. And then the product was confirmed to have a discontinuous-micellar cubic liquid-crystal phase as the outer phase.

## Claims

1. A cosmetic composition comprising:
(A) an alkyl ethylene oxide surfactant having an HLB of 8 to 13,
(B) 10 to 40 mass % of an oil component, and
(C) water,
wherein said composition is a discontinuous-micellar cubic liquid-crystal phase.

2. The cosmetic composition according to claim 1, wherein the mass ratio of the component (A) and the component (C) is (A):(C) = 20:80 to 70:30.

3. The cosmetic composition according to claim 1 or 2, wherein said cosmetic composition is a cleansing composition.

4. The cosmetic composition according to claim 3, wherein said cleansing composition is a skin or hair cleansing composition.

5. The cosmetic composition according to claim 3, wherein said cleansing composition is a makeup cleansing base formula.

6. The cosmetic composition according to any of claims 1 to 5, further comprising (D) a polyhydric alcohol and/or a saccharide.

7. The cosmetic composition according to claim 6, wherein the component (D) is polyethylene glycol and/or sorbitol.

8. A cosmetic composition, wherein a discontinuous-micellar cubic liquid-crystal phase comprising:
(A) an alkyl ethylene oxide surfactant having an HLB of 8 to 13,
(B₁) an oil component, and
(C) water
is the outer phase and
(B₂) an oil phase comprising an oil component that is different from the oil component (B₁) captured in said discontinuous-micellar cubic liquid-crystal phase is contained as the inner phase.

9. The cosmetic composition according to claim 8, wherein the total mass ((B₁) + (B₂)) of the oil component (B) in said cosmetic composition is 20 to 80 mass % with respect to the total cosmetic composition.

10. The cosmetic composition according to claim 9 or 10, wherein the mass ratio of the component (A) and the component (C) is (A):(C)=20:80 to 70:30.

11. The cosmetic composition according to any of claims 8 to 10, wherein said cosmetic composition is a cleansing composition.

12. The cosmetic composition according to claim 11, wherein said cleansing composition is a skin or hair cleansing composition.

13. The cosmetic composition according to claim 11, wherein said cleansing composition is a makeup cleansing base formula.

14. The cosmetic composition according to any of claims 8 to 13, further comprising (D) a polyhydric alcohol and/or a saccharide.

15. The cosmetic composition according to claim 14, wherein the component (D) comprises one or more selected from the group consisting of 1,3-butylene glycol, dipropylene glycol, glycerin, polyethylene glycol, and sorbitol.
